# FASCICULE DE BREVET EUROPEEN

(11) **EP 1 746 948 B1**
(45) Date de publication et mention de la délivrance du brevet: **25.02.2015**
(21) Numéro de dépôt: 05771224.2
(22) Date de dépôt: 13.05.2005
(51) Int. Cl.: A61B 17/70

(54) **CALE INTERVERTEBRALE POUR VERTEBRES CERVICALES**
ZWISCHENWIRBELKEILSTÜCK FÜR HALSWIRBEL
INTERVERTEBRAL WEDGE FOR CERVICAL VERTEBRAE

(30) Priorité: 17.05.2004 FR 0405333
(43) Date de publication de la demande: 31.01.2007
(73) Titulaire: ZIMMER SPINE, 33000 Bordeaux (FR)
(72) Inventeur: PASQUET, Denis, F-13100 Aix-en-Provence (FR); LE COUEDIC, Régis, F-78570 Andresy (FR)
(74) Mandataire: Venner Shipley LLP
(86) Numéro de dépôt international: PCT/FR2005/001202
(87) Numéro de publication internationale: WO 2005/122924

(56) Documents cités:
- WO-A-2004/084743
- FR-A- 2 799 640
- PATENT ABSTRACTS OF JAPAN vol. 2003, no. 07, 3 juillet 2003 (2003-07-03) -& JP 2003 079649 A (PENTAX CORP; HASE HITOSHI), 18 mars 2003 (2003-03-18)
- PATENT ABSTRACTS OF JAPAN vol. 2000, no. 06, 22 septembre 2000 (2000-09-22) -& JP 2000 070277 A (KYOCERA CORP), 7 mars 2000 (2000-03-07)
- PATENT ABSTRACTS OF JAPAN vol. 1999, no. 09, 30 juillet 1999 (1999-07-30) -& JP 11 089854 A (KOBE STEEL LTD), 6 avril 1999 (1999-04-06)

## Description

La présente invention a pour objet une cale intervertébrale destinée à être mise en place entre deux vertèbres cervicales adjacentes, et un ensemble de cales intervertébrales comprenant deux cales intervertébrales du type précité.

Le rachis humain est formé de vingt-quatre vertèbres vraies, placées les unes au-dessus des autres et reliées par des fibrocartilages appelés disques intervertébraux. Ces vertèbres se divisent en trois groupes : 7 vertèbres cervicales, 12 vertèbres dorsales, et 5 vertèbres lombaires. Les vertèbres ont des formes différentes suivant les trois niveaux (cervical, dorsal et lombaire) du rachis, mais elles gardent certains caractères généraux. Toute vertèbre comprend :
- une partie antérieure renflée, le corps vertébral ;
- un arc osseux à concavité antérieure, généralement appelé arc postérieur ou arc neural, circonscrivant avec la face postérieure du corps vertébral un orifice que traverse la moelle épinière : le trou rachidien, cet arc étant formé de chaque côté par des pédicules en avant et par des lames vertébrales en arrière ;
- une saillie médiane et postérieure, l'apophyse épineuse.

Au cours de la vie d'un individu, il arrive qu'un disque intervertébral, pour diverses raisons, finisse par être endommagé. Dans ce cas, les contraintes qui s'exercent sur ce disque et qui résultent des déplacements relatifs entre les deux vertèbres entourant le disque, sont à l'origine de douleurs. Aussi, pour soulager ces douleurs, on cherche à bloquer les deux vertèbres l'une par rapport à l'autre.

On connaît déjà de nombreuses cales vertébrales destinées à être mises en place entre deux vertèbres dorsales ou lombaires adjacentes. Ces cales présentent généralement deux évidements longitudinaux ménagés respectivement sur leurs faces supérieure et inférieure, destinés à recevoir chacun l'apophyse épineuse de l'une des deux vertèbres. Un exemple de ce type de cale est donné dans la demande de brevet français rendue publique sous le numéro de publication FR 2 799 640.

Si ce type de cale connu se révèle bien adapté aux niveaux dorsal et lombaire, il s'avère néanmoins impossible de l'utiliser au niveau des vertèbres cervicales, en partie pour les raisons suivantes.

D'une part, le rachis présente au niveau cervical une convexité antérieure, la lordose cervicale, qui rapproche les apophyses épineuses des vertèbres cervicales. Ainsi, ces apophyses sont si proches l'une de l'autre (certaines apophyses se touchent) qu'il est impossible d'installer une cale entre elles.

D'autre part, alors qu'au niveau dorsal ou lombaire, les mouvements relatifs de deux vertèbres adjacentes consistent principalement en un écartement ou un rapprochement de ces vertèbres, ces mouvements étant respectivement provoqués par la flexion ou l'extension du rachis, au niveau cervical des mouvements plus complexes entrent en jeu. Ainsi, les mouvements de rotation d'une vertèbre par rapport à une autre, provoqués par la torsion du rachis, ou les mouvements provoqués par l'inflexion latérale du rachis (i.e. l'écartement latéral du rachis par rapport au plan sagittal) et qui se décomposent en une rotation et un écartement asymétrique d'une vertèbre par rapport à une autre, sont nettement plus marqués au niveau cervical qu'au niveau dorsal ou lombaire.

L'invention a donc pour objet une cale intervertébrale destinée à être mise en place entre deux vertèbres cervicales adjacentes, supérieure et inférieure, afin de limiter le déplacement relatif de ces vertèbres. La demande de brevet japonaise 2003-79649 divulgue les caractéristiques de la préambule de la présente revendication indépendante.

Les 7 vertèbres cervicales sont généralement numérotées en partant du haut du rachis, et désignées C1 à C7. La vertèbre C1 est également appelée l'atlas et la vertèbre C2 l'axis. La région intervertébrale altlanto-axiale (située entre les vertèbres C1 et C2) est anatomiquement très, différente des régions intervertébrales inférieures, en raison de la forme particulière de la vertèbre C1.

La cale de l'invention a été conçue plus particulièrement pour être mise en place entre deux vertèbres cervicales adjacentes, autres que l'atlas et l'axis, c'est-à-dire entre deux vertèbres adjacentes choisies parmi C2 à C7.

Selon son mode de réalisation le plus général, la cale de l'invention comprend un corps de cale et des moyens de maintien pour maintenir ce corps de cale en position contre lesdites vertèbres cervicales adjacentes, supérieure et inférieure, ledit corps de cale comprenant :
- une pièce d'écartement avec deux extrémités opposées, une portion de la première extrémité étant susceptible d'être insérée entre le bord supérieur de la lame de la vertèbre cervicale inférieure et le bord inférieur de la lame de la vertèbre cervicale supérieure;
- deux ailes, supérieure et inférieure, liées par leur base à la deuxième extrémité de la pièce d'écartement et qui s'étendent de chaque côté de cette dernière, chaque aile présentant une face antérieure tournée vers la pièce d'écartement et une face postérieure, les faces antérieures des ailes supérieure et inférieure étant respectivement susceptibles de venir en appui contre les parties postérieures des lames des vertèbres supérieure et inférieure.

La forme particulière de cette cale lui permet de s'adapter à la morphologie de la région cervicale pour remplir ses fonctions. En particulier, la pièce d'écartement maintient les lames des vertèbres cervicales supérieure et inférieure à distance l'une de l'autre, ce qui permet de soulager le disque intervertébral de certaines contraintes en compression et, en particulier, d'éviter tout « pincement » lié à l'extension du rachis.

D'autre part, les ailes supérieure et inférieure, en prenant appui contre les lames des vertèbres, garantissent une position stable du corps de cale part rapport à ces dernières.

Lesdits moyens de maintien pour maintenir le corps de cale en position contre lesdites vertèbres, comprennent une sangle présentant deux extrémités et des moyens de fixation autobloquants permettant de solidariser au moins une des extrémités de la sangle au corps de cale, ladite sangle étant destinée à être serrée autour des lames des vertèbres supérieure et inférieure.

Les moyens de maintien étreignent donc le corps de la cale et les lames vertébrales, ce qui permet de maintenir la pièce d'écartement enfoncée entre lesdites lames et d'empêcher cette pièce de sortir de son logement. Ces moyens garantissent donc le maintien en position de la cale une fois que celle-ci a été implantée, quels que soient les mouvements du patient ayant subi l'implantation.

En outre, lesdits moyens de maintien empêchent les vertèbres de s'écarter l'une de l'autre de manière à préserver le disque intervertébral de toute contrainte, en particulier lors de la flexion du rachis.

Dans la pratique, pour limiter correctement les déplacements relatifs entre deux vertèbres adjacentes, il est nécessaire d'utiliser deux cales intervertébrales du type de celle précédemment décrite. Pour cette raison, l'invention a également pour objet un ensemble de cales intervertébrales, caractérisé en ce qu'il comprend une première et une deuxième cales intervertébrales du type de celle précédemment décrite, la première cale étant destinée à être disposée entre les lames des vertèbres cervicales supérieure et inférieure, situées d'un même côté des apophyses épineuses de ces vertèbres et la deuxième cale étant destinée à être disposée entre les lames des vertèbres cervicales supérieure et inférieure situées de l'autre côté desdites apophyses épineuses.

Cette combinaison de deux cales permet de bloquer tout mouvement relatif entre les vertèbres concernées : écartement, rapprochement ou rotation, et ce quel que soit le mouvement général du rachis : extension, flexion, torsion, ou inflexion latérale.

Les différentes caractéristiques et principaux avantages de l'invention seront mieux compris à la lecture des modes de réalisation particuliers de cales intervertébrales donnés à titre d'exemple et illustrés par les figures suivantes :
- la figure 1 est une vue extérieure en perspective d'un premier type de cale selon l'invention ;
- la figure 2 est une vue éclatée, en coupe selon le plan II-II, de la cale représentée figure 1 ;
- la figure 2a est une vue en coupe de la cale des figures 1 et 2 une fois que celle-ci est mise en place entre les lames de deux vertèbres cervicales adjacentes ;
- la figure 3 est une vue éclatée, en coupe, d'un deuxième type de cale selon l'invention ;
- la figure 3a est une vue en coupe de la cale de la figure 3 une fois que celle-ci est mise en place entre deux vertèbres ;
- la figure 4 est une vue extérieure en perspective d'un ensemble de deux cales du type de celle représentée figures 3 et 3a ; et
- la figure 5 est une vue de dessus de l'ensemble de la figure 4 une fois que celui-ci est mis en place entre deux vertèbres.

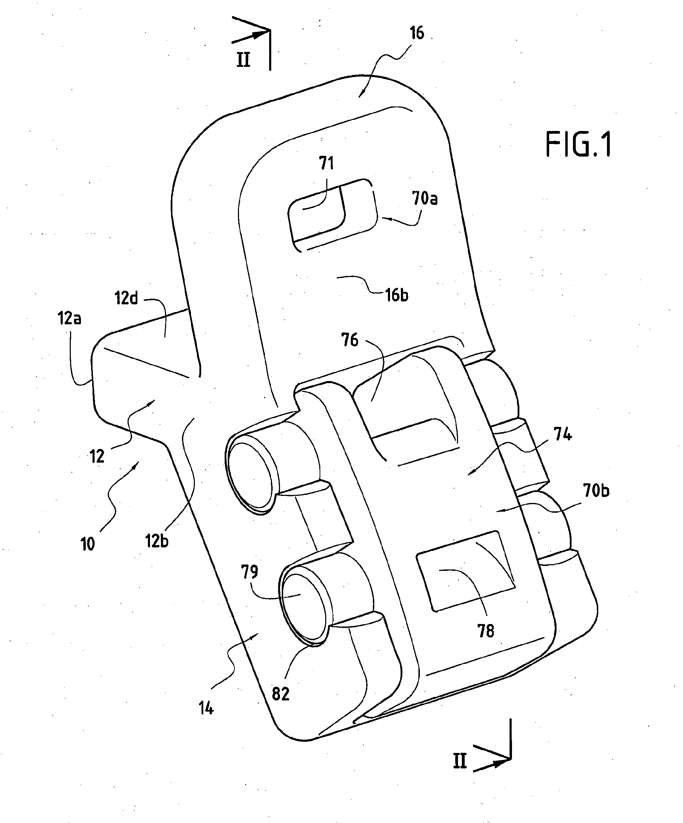

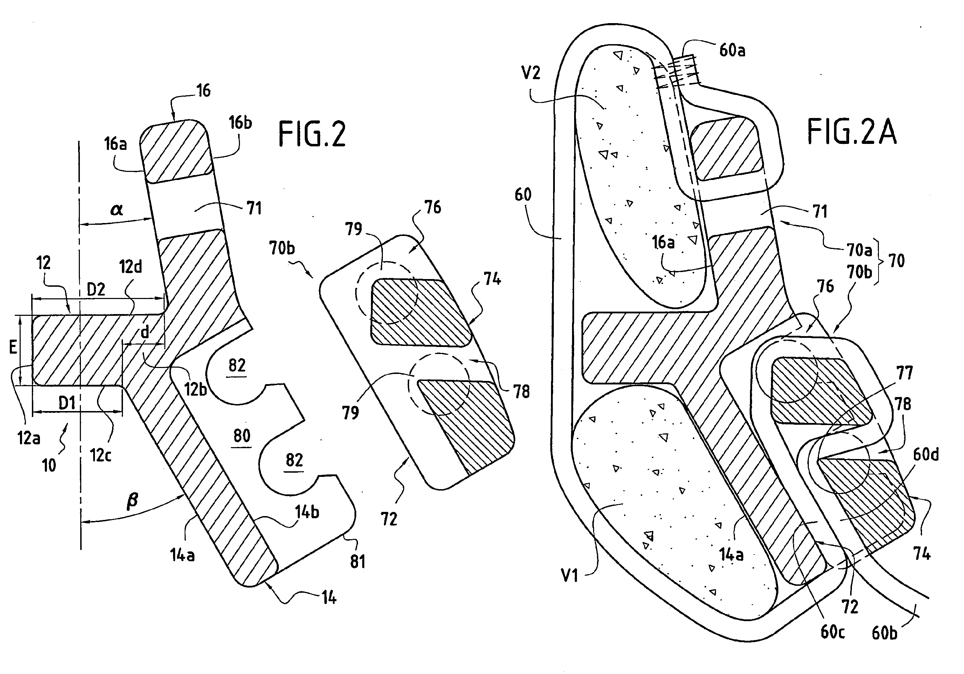

Selon un premier mode de réalisation de l'invention et en référence aux figures 1, 2 et 2a, la cale comprend un corps de cale 10 comprenant lui-même une pièce d'écartement 12 qui présente une première extrémité 12a, distale, et une deuxième extrémité 12b, proximale, à partir de laquelle s'étendent de chaque côté de la pièce 12, deux ailes supérieure 16 et inférieure 14. Les ailes 14 et 16 présentent chacune respectivement une face antérieure 14a, 16a tournée vers la pièce d'écartement 12 et une face postérieure 14b, 16b opposée à leur face antérieure.

Le corps de cale 10 est symétrique par rapport à son plan médian II-II représenté figure 1, ce plan étant parallèle à la direction selon laquelle on mesure la distance entre l'extrémité distale 12a et l'extrémité proximale 12b de la pièce d'écartement 12.

La pièce d'écartement 12 du corps de cale 10 est destinée à être insérée entre deux vertèbres cervicales supérieure V2 et inférieure V1. Dans l'exemple représenté, cette pièce 12 présente deux faces supérieure 12d et inférieure 12c opposées, sensiblement parallèles entre elles. La face supérieure 12d est destinée à venir au contact du bord inférieur de la lame de la vertèbre supérieure V2 tandis que la face inférieure 12c est destinée à venir au contact du bord supérieur de la lame de la vertèbre inférieure V1.

Pour écarter les vertèbres V1 et V2 d'une certaine distance, on va bien entendu jouer sur l'épaisseur E, qui sépare les faces 12c et 12d, de la pièce d'écartement 12. Avantageusement, on choisit cette épaisseur E comprise entre 2 et 8 millimètres. En effet, on a pu constater qu'une telle épaisseur permettait de soulager efficacement le disque intervertébral situé entre les vertèbres V1 et V2.

Une autre caractéristique de la pièce d'écartement 12 est la distance D1 entre sa première extrémité 12a, distale, et la base de l'aile inférieure 14. Cette distance D1 détermine la profondeur de pénétration de la pièce 12 entre les vertèbres V1 et V2. Aussi, la distance D1 doit être suffisamment importante pour que la cale reste en position entre les vertèbres V1 et V2, quels que soient les mouvements de celles-ci. La distance D1 doit également être suffisamment faible pour que l'extrémité 12a n'avance pas trop à l'intérieur du trou rachidien. En effet, il convient de limiter les contacts trop marqués entre cette extrémité 12a et la moelle épinière, qui pourraient être à l'origine de douleurs chez le patient. On notera néanmoins que la moelle épinière au niveau cervical est protégée par un ligament appelé ligament jaune, de sorte que de légers contacts entre ce ligament et l'extrémité 12a peuvent exister. Dans la pratique, on peut choisir la distance D1 comprise entre 2 et 6 millimètres (mm).

On définit la distance D2 comme la distance séparant la première extrémité 12a de la pièce d'écartement 12 et la base de l'aile supérieure, et la distance d comme étant la différence entre D2 et D1 (D2 - D1).

Avantageusement, la distance D2 est choisie pour que d soit comprise dans un intervalle de distance qui permette au corps de cale de s'adapter au décalage naturel existant entre les vertèbres cervicales. En effet, en raison de la lordose cervicale, le bord supérieur de la lame de la vertèbre inférieure, est en retrait par rapport au bord inférieur de la lame de la vertèbre supérieure adjacente. Ainsi, avantageusement, la distance d= D2 - D1 est comprise entre 0 et 5 mm.

D'autre part, la lordose cervicale a également pour conséquence l'inclinaison des lames des vertèbres cervicales entre elles. Avantageusement, pour tenir compte de cette inclinaison, la face antérieure 16a de l'aile supérieure 16 forme avec la face antérieure 14a de l'aile inférieure 14 un angle (β - α) compris entre 0 et 60°. Dans l'exemple représenté, l'angle (β - α) se mesure sans difficulté, les faces antérieures 16a et 14a étant sensiblement planes. Si tel n'était pas le cas, cet angle (β - α) serait mesuré entre les plans moyens des faces 16a et 14a.

La cale intervertébrale de l'invention comprend, en outre, des moyens de maintien pour maintenir le corps de cale 10 en position contre les vertèbres V1 et V2. Un premier type de moyens de maintien est représenté sur les figures 1 à 3.

Ces moyens de maintien comprennent une sangle 60 présentant deux extrémités 60a et 60b, et des moyens de fixation 70 qui permettent de solidariser les extrémités 60a et 60b au corps de cale 10. Les moyens de fixation 70 comprennent des premiers moyens de fixation 70a situés sur l'une des ailes du corps 10 de cale, ici l'aile supérieure 16, et des deuxièmes moyens de fixation autobloquants 70b situés sur l'autre aile du corps de cale 10, ici l'aile inférieure 14. Les premiers et deuxièmes moyens de fixation 70a et 70b permettent respectivement de fixer les premières et deuxièmes extrémités 60a et 60b de la sangle 60 au corps de cale 10.

Les premiers moyens de fixation 70a comprennent une fente 71 ménagée dans l'aile supérieure 16, à travers laquelle une portion d'extrémité 60a de la sangle est passée avant que l'extrémité 60a ne soit cousue (ou fixée par tout autre moyen) sur la sangle elle-même, de manière à former une boucle qui entoure la partie de l'aile supérieure 16 située au-dessus de la fente 71.

La deuxième extrémité de la sangle 60b, quant à elle, est passée puis tirée au travers des deuxièmes moyens de fixation 70b pour serrer la sangle 60 autour des lames des vertèbres supérieure et inférieure V2 et V1. Les deuxièmes moyens de fixation sont réalisés de manière à susciter des forces de frottement qui s'opposent au desserrage de ladite sangle. Pour cette raison, on parle de moyens de fixation autobloquants. Dans l'exemple représenté figures 1, 2 et 2a, les deuxièmes moyens de fixation 70b sont amovibles par rapport au corps de la cale 10 et sont susceptibles d'être rapportés sur la face postérieure 14b de l'aile inférieure 14.

Les deuxièmes moyens de fixation 70b présentent une face antérieure 72 et une face postérieure 74 (opposée à la face 72) et sont traversés par une première 76 et une deuxième fente 78 qui débouchent sur lesdites faces 72, 74. Ces fentes, sans être nécessairement strictement parallèles entre elles, sont inclinées dans le même sens par rapport aux faces 72 et 74.

La deuxième extrémité 60b de la sangle 60 est passée dans les fentes 76, 78 de la manière suivante : la sangle est rentrée dans la première fente 76, du côté de la face antérieure 72, et ressortie du côté de la face postérieure, puis rentrée dans la deuxième fente 78, du côté de la face postérieure 74, et ressortie du côté de la face antérieure 72. Ainsi, une portion de la sangle 60c située en amont de la première fente 76 recouvre une portion de sangle 60d située en aval de la deuxième fente 78.

Un logement 80 est ménagé sur la face postérieure 14b de l'aile 14 du corps de cale 10 pour recevoir les deuxièmes moyens de fixation 70b. Des ouvertures 82 sont ménagées dans les bords latéraux 81 de ce logement 80 et sont aptes à coopérer par clipsage avec des tétons 79 présents sur les bords latéraux des deuxièmes moyens de fixation 70b. Ainsi, les deuxièmes moyens de fixation 70b peuvent être rapportés sur le corps de cale 10.

Une fois les deuxièmes moyens de fixation rapportés sur le corps de cale 10, les portions de sangle 60c et 60d qui se recouvrent sont serrées l'une contre l'autre de sorte que des forces de frottement significatives existent entre ces portions. D'autre part, la deuxième fente 78 forme avec la face postérieure 72 des moyens de fixation 70b un angle aigu de sorte que l'arête 77 située à la sortie (dans le sens du passage de la sangle 60) de la deuxième fente 78 est vive. Les forces de frottement entre cette arête 77 et la sangle 60 sont également significatives. Une fois que la sangle 60 est serrée autour des lames des vertèbres V1 et V2, les forces de frottement précitées s'opposent au desserrage de celle-ci.

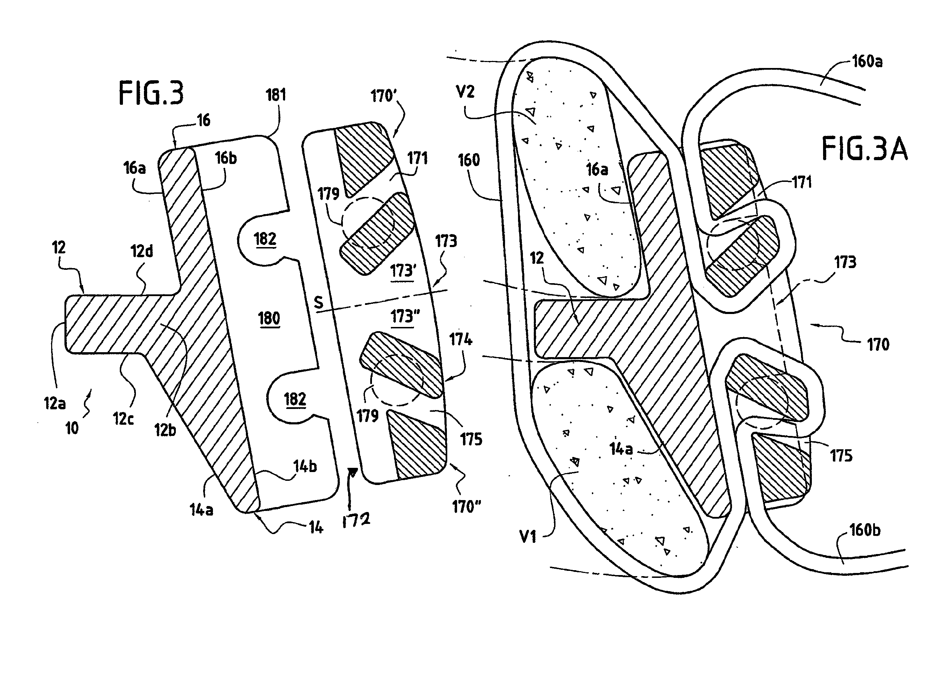

Les figures 3, 3A, 4 et 5 représentent un deuxième mode de réalisation de cale selon l'invention.

Cette cale comprend un corps de cale 10 sensiblement identique à celui précédemment décrit. Pour cette raison, les parties du corps de cale des figures 3 à 5, analogues à celles du corps de cale des figures 1 et 2, sont affectées des mêmes références numériques. La différence majeure entre ces deux corps de cales réside dans le fait que, dans le deuxième exemple, les faces postérieures 16a et 14a des ailes supérieure 16 et inférieure 14 sont coplanaires.

En revanche, dans ce deuxième mode de réalisation, la cale comprend des moyens de maintien pour maintenir le corps de cale 10 en position contre les vertèbres V1 et V2, différents des moyens de maintien décrits jusqu'à présent.

Ces moyens de maintien comprennent une sangle 160 présentant deux extrémités 160a, 160b et des moyens de fixation autobloquants 170 permettant de solidariser ces deux extrémités 160a, 160b au corps de cale 10. Ces moyens de fixation autobloquants 170 s'étendent sur les faces postérieures 16b, 14b des ailes supérieure 16 et inférieure 14. Cette disposition est facilitée, dans l'exemple représenté, par le fait que les deux faces postérieures 16b et 14b sont coplanaires. Ces moyens 170 pourraient néanmoins être situés uniquement sur une seule des faces postérieures 16b, 14b.

Les moyens de fixation autobloquants 170 sont amovibles par rapport au corps de cale 10 et sont dotés sur leurs bords latéraux, de tétons 179 (2 tétons par bord latéral) aptes à coopérer par clipsage avec des ouvertures 182 ménagées dans les bords latéraux 181 de la partie postérieure du corps de cale 10. Ces bords latéraux 181 définissent avec les faces postérieures 14b et 16b des ailes 14 et 16, un logement 180 apte à recevoir lesdits moyens de fixation autobloquants 170.

Ces moyens de fixation 170 présentent une face antérieure 172 et une face postérieure 174 et sont traversés par trois fentes transversales successives 171, 173, et 175 qui débouchent sur les faces 172 et 174. La première fente 171 et la troisième 175 sont inclinées l'une vers l'autre, tandis que la deuxième fente 173, centrale, est une fente en V. Ces moyens de fixation 170 présentent un plan de symétrie qui correspond au plan médian de la fente 173 en V. Ce plan de symétrie divise les moyens de fixation 170 en deux demi ensembles 170' et 170", la fente 173 étant elle-même divisée en deux demi fentes 173' et 173".

La structure d'un demi ensemble 170' ou 170" est analogue à la structure des moyens de fixation 70b décrits et représentés figures 1, 2 et2A. En particulier, les deux fentes 171 et 173', ou 175 et 173", équivalent respectivement aux première 78 et deuxième fente 76 des moyens 70b. Ainsi, les extrémités de sangle 160a et 160b sont respectivement passées dans les systèmes de fentes 171 et 173', et 175 et 173", de la même manière que l'extrémité 60b est passée au travers du système de fentes 76 et 78, de sorte que des forces de frottement permettent des s'opposer au desserrage de la sangle 160 une fois que celle-ci est serrée autour des vertèbres V1 et V2.

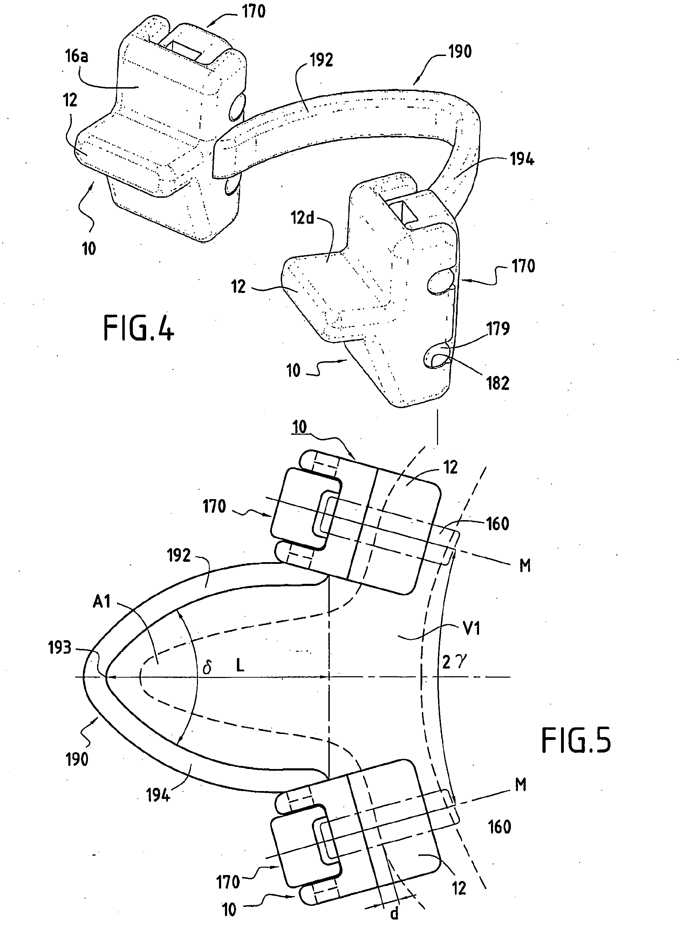

En référence aux figures 4 et 5, on va maintenant décrire un exemple d'ensemble de cales intervertébrales, comprenant deux cales du type de celle représentée sur les figures 3 et3A, et un élément de liaison 190 qui relie les cales entre elles. De manière générale l'élément de liaison 190 présente une certaine rigidité et sa forme est choisie telle que cet élément 190 contourne les apophyses épineuses A1, A2, des vertèbres adjacentes inférieure V1 et supérieure V2.

Dans l'exemple, ledit élément de liaison 190 a la forme générale d'un V et présente deux branches incurvées, écartées l'une de l'autre, dont les extrémités libres sont fixées aux première et deuxième cales 170. La longueur des branches, leur courbure, et leur angle d'écartement δ sont choisis tels que l'élément de jonction 190 contourne les apophyses A1, A2 des vertèbres V1, V2 ainsi, avantageusement, la distance L entre les extrémités libres des branches et le point de jonction 193 desdites branches, est comprise entre 10 et 40 millimètres, et l'angle d'écartement δ desdites branches est compris entre 10 et 60°.

La forme de l'élément de liaison 190 et la manière dont il est fixé aux cales 170 sont tels que les plans médians M des pièces d'écartement 12 des cales 170, ces plans M étant orientés dans le sens de l'épaisseur des pièces d'écartement 12, définissent entre eux un dièdre d'angle rectiligne 2γ (on notera que le plan M correspond au plan II-II de la figure 1). L'angle 2γ est choisi de sorte que, lorsqu'on installe l'une des cales 170 entre les lames des vertèbres V1 et V2 situées d'un même côté des apophyses épineuses A1, A2, de ces vertèbres, l'autre cale 170 viennent se positionner (ou tout au moins tendent à venir à se positionner) au bon endroit entre les lames des vertèbres V1 et V2 situées de l'autre côté des apophyses A1 et A2. Avantageusement, l'angle 2γ est compris entre 0 et 60°.

Cet ensemble de cales facilite donc le travail du chirurgien lors de la mise en place des cales 170, d'une part parce que le chirurgien n'a à manipuler qu'un seul ensemble au lieu de deux cales séparées, et ensuite parce que le positionnement de l'une des cales 170 guide le positionnement de l'autre cale.

La structure des cales et de l'ensemble de cales selon l'invention étant bien comprise, nous allons maintenant décrire une méthode de mise en place de ces cales entre deux vertèbres cervicales adjacentes V2 et V1.

Une telle méthode comprend les étapes suivantes :
- on ménage une voie d'accès à la partie postérieure du rachis, au niveau desdites vertèbres cervicales V1 et V2 ;
- on installe le corps de cale d'une première cale du type de celle précédemment décrite, entre les lames desdites vertèbres cervicales, situées d'un même coté des apophyses épineuses A2, A1 de ces vertèbres ;
- on solidarise le corps de cale de cette première cale auxdites vertèbres V2, V1 et on le maintient en position contre ces vertèbres à l'aide des moyens de maintien du type de ceux précédemment décrits ;
- on installe le corps de cale d'une deuxième cale, identique à la première, entre les lames desdites vertèbres cervicales V2 et V1, situées de l'autre coté des apophyses épineuses A2, A1 de ces vertèbres ; et
- on solidarise le corps de cale de cette deuxième cale auxdites vertèbres V2, V1 et on le maintient en position contre ces vertèbres à l'aide des moyens de maintien du type de ceux précédemment décrits.

Les première et deuxième cales précitées peuvent être indépendantes l'une de l'autre ou appartenir à un ensemble de cale du type de celui précédemment décrit.

## Revendications

1. Cale intervertébrale destinée à être mise en place entre deux vertèbres cervicales adjacentes (V2, V1), supérieure et inférieure, comprenant un corps de cale (10) et des moyens pour maintenir ce corps de cale (10) en position contre lesdites vertèbres, ledit corps de cale comprenant :
- une pièce d'écartement (12) avec des première et deuxième extrémités opposées (12a, 12b), une portion de la première extrémité (12a) étant susceptible d'être insérée entre le bord supérieur de la lame de la vertèbre cervicale inférieure (V1) et le bord inférieur de la lame de la vertèbre cervicale supérieure (V2);
- deux ailes (16, 14), supérieure et inférieure, liées par leur base à la deuxième extrémité (12b) de la pièce d'écartement (12) et qui s'étendent de chaque côté de cette dernière, chaque aile présentant une face antérieure (16a, 14a) tournée vers la pièce d'écartement (12) et une face postérieure (16b, 14b), les faces antérieures (16a, 14a) des ailes supérieure et inférieure étant respectivement susceptibles de venir en appui contre les parties postérieures des lames des vertèbres supérieure et inférieure (V2, V1);
**caractérisée en ce que** lesdits moyens pour maintenir en position le corps de cale comprennent une sangle (60 ; 160) présentant deux extrémités (60a, 60b ; 160a, 160b) et des moyens de fixation autobloquants (70b, 170) permettant de solidariser au moins une des extrémités de la sangle au corps de cale (10), ladite sangle (60 ; 160) étant destinée à être serrée autour des lames des vertèbres supérieure et inférieure (V2, V1).

2. Cale intervertébrale selon la revendication 1, **caractérisée en ce que** lesdits moyens pour maintenir en position le corps de cale comprennent des premiers moyens de fixation (70a) situés sur l'une des ailes du corps de cale (10), pour fixer une première extrémité (60a) de ladite sangle (60) au corps de cale, et des deuxièmes moyens de fixation autobloquants (70b), situés sur l'autre aile du corps de cale, au travers desquels la deuxième extrémité (60b) de la sangle est passée puis tirée pour serrer la sangle autour des lames des vertèbres supérieure et inférieure (V2, V1), les deuxièmes moyens de fixation (70b) étant réalisés de manière à susciter des forces de frottement qui s'opposent au desserrage de ladite sangle (60). (70b) étant réalisés de manière à susciter des forces de frottement qui s'opposent au desserrage de ladite sangle (60).

3. Cale intervertébrale selon la revendication 1, **caractérisée en ce que** lesdits moyens de fixation autobloquants (170) sont situés sur la face postérieure de l'aile supérieure (16) et/ou de l'aile inférieure (14), les première et deuxième extrémités de la sangle (160a, 160b) étant passées dans ces moyens de fixation (170), puis tirées pour serrer la sangle autour des lames des vertèbres supérieure et inférieure (V2, V1), ces moyens de fixation autobloquants étant réalisés de manière à susciter des forces de frottement qui s'opposent au desserrage de ladite sangle (160).

4. Cale intervertébrale selon l'une quelconque des revendications 1 à 3, **caractérisée en ce que** les faces postérieures (16b, 14b) des ailes supérieure et inférieure sont coplanaires.

5. Cale intervertébrale selon l'une quelconque des revendications 1 à 4, **caractérisée en ce que** lesdits moyens de fixation auto-bloquants (70b, 170) sont amovibles par rapport au corps de la cale (10) et rapportés sur la face postérieure de l'aile supérieure (16) et/ou de l'aile inférieure (14).

6. Ensemble de cales intervertébrales, **caractérisé en ce qu'**il comprend une première et une deuxième cale intervertébrale selon l'une quelconque des revendications précédentes, la première cale étant destinée à être disposée entre les lames des vertèbres cervicales supérieure et inférieure (V2, V1), situées d'un même coté des apophyses épineuses (A2, A1) de ces vertèbres et la deuxième cale étant destinée à être disposée entre les lames des vertèbres cervicales supérieure et inférieure situées de l'autre coté desdites apophyses épineuses (A2, A1).

7. Ensemble de cales intervertébrales selon la revendication 6, **caractérisé en ce qu'**il comprend en outre un élément de liaison (190) qui relie les cales entre elles.

8. Ensemble de cales intervertébrales selon la revendication 7, **caractérisé en ce que** la forme dudit élément de liaison (190) est choisie telle que cet élément contourne les apophyses épineuses (A2, A1) desdites vertèbres (V2, V1).

9. Ensemble de cales intervertébrales selon la revendication 8, **caractérisé en ce que** ledit élément de liaison (190) a la forme générale d'un V et présente deux branches (192, 194) incurvées écartées l'une de l'autre dont les extrémités libres sont fixées aux première et deuxième cales, la longueur des branches, leur courbure, et leur angle d'écartement (5) étant choisis tels que l'élément de jonction contourne les apophyses épineuses (A2, A1) desdites vertèbres.

10. Ensemble de cales intervertébrales selon la revendication 9, **caractérisé en ce que** la distance (L) entre les extrémités libres des branches (192, 194) et le point de jonction (193) desdites branches est comprise entre 10 et 40 mm, et **en ce que** l'angle d'écartement (δ) desdites branches est compris entre 10 et 60°.

## Patentansprüche

1. Zwischenwirbelkeil, der dazu bestimmt ist, zwischen zwei benachbarten Halswirbeln (V2, V1), einem oberen und einem unteren, angeordnet zu werden, umfassend einen Keilkörper (10) und Mittel, um diesen Keilkörper (10) in Position gegen die Wirbel zu halten, wobei der Keilkörper Folgendes umfasst:
- ein Abstandsstück (12) mit einem ersten und einem zweiten Ende (12a, 12b), die einander gegenüberliegen, wobei ein Teil des ersten Endes (12a) zwischen dem oberen Rand der Bogenplatte des unteren Halswirbels (V1) und dem unteren Rand der Bogenplatte des oberen Halswirbels (v2) einsetzbar ist;
- zwei Flügel (16, 14), einen oberen und einen unteren, die über ihre Basis mit dem zweiten Ende (12b) des Abstandsstücks (12b) verbunden sind und die sich an jeder Seite davon erstrecken, wobei jeder Flügel eine vorderseitige Fläche (16a, 14a), die zu dem Abstandsstück (12) hin gedreht ist, und eine rückseitige Fläche (16b, 14b) aufweist, wobei die vorderseitigen Flächen (16a, 14a) des oberen und unteren Flügels jeweils geeignet sind, gegen die rückseitigen Teile der Bogenplatten des oberen und unteren Wirbels (V2, V1) zur Auflage zu kommen;
**dadurch gekennzeichnet, dass** die Mittel, um den Keilkörper in Position zu halten, einen Gurt (60; 160) umfassen, der zwei Enden (60a, 60b ; 160a, 160b) und selbsthemmende Befestigungsmittel (70b, 170) aufweist, die es ermöglichen, mindestens eines der Enden des Gurtes fest mit dem Keilkörper (10) zu verbinden, wobei der Gurt (60; 160) dazu bestimmt ist, um die Bogenplatten des oberen und unteren Wirbels (V2, V1) herum festgespannt zu werden.

2. Zwischenwirbelkeil nach Anspruch 1, **dadurch gekennzeichnet, dass** die Mittel, um den Keilkörper in Position zu halten, erste Befestigungsmittel (70a) umfassen, die sich an dem einen der Flügel des Keilkörpers (10) befinden, um ein erstes Ende (60a) des Gurtes (60) mit dem Keilkörper zu verbinden, und zweite selbsthemmende Befestigungsmittel (70b) umfassen, die sich an dem anderen Flügel des Keilkörpers befinden, durch die hindurch das zweite Ende (60b) des Gurtes geführt und dann angezogen wird, um den Gurt um die Bogenplatten des oberen und unteren Wirbels (V2, V1) herum festzuspannen, wobei die zweiten Befestigungsmittel (70b) derart ausgebildet sind, dass sie Reibungskräfte hervorrufen, die das Lösen des Gurtes (60) verhindern.

3. Zwischenwirbelkeil nach Anspruch 1, **dadurch gekennzeichnet, dass** die selbsthemmenden Befestigungsmittel (170) sich an der rückseitigen Fläche des oberen Flügels (16) und/oder des unteren Flügels (14) befinden, wobei das erste und zweite Ende des Gurtes (160a, 160b) in diese Befestigungsmittel (170) geführt und dann angezogen werden, um den Gurt um die Bogenplatten des oberen und unteren Wirbels (V2, V1) herum festzuspannen, wobei diese selbsthemmenden Befestigungsmittel derart ausgebildet sind, dass sie Reibungskräfte hervorrufen, die das Lösen des Gurtes (160) verhindern.

4. Zwischenwirbelkeil nach einem der Ansprüche 1 bis 3, **dadurch gekennzeichnet, dass** die rückseitigen Flächen (16b, 14b) des oberen und unteren Flügels koplanar sind.

5. Zwischenwirbelkeil nach einem der Ansprüche 1 bis 4, **dadurch gekennzeichnet, dass** die selbsthemmenden Befestigungsmittel (70b, 170) relativ zu dem Keilkörper (10) entfernbar sind und an der rückseitigen Fläche des oberen Flügels (16) und/oder des unteren Flügels (14) angebracht sind.

6. Zwischenwirbelkeilanordnung, **dadurch gekennzeichnet, dass** sie einen ersten und einen zweiten Zwischenwirbelkeil nach einem der vorhergehenden Ansprüche umfasst, wobei der erste Keil dazu bestimmt ist, zwischen den Bogenplatten des oberen und unteren Halswirbels (V2, V1) angeordnet zu werden, die sich auf einer gleichen Seite der Dornfortsätze (A2, A1) dieser Wirbel befinden, und wobei der zweite Keil dazu bestimmt ist, zwischen den Bogenplatten des oberen und unteren Halswirbels angeordnet zu werden, die sich auf der anderen Seite der Dornfortsätze (A2, A1) befinden.

7. Zwischenwirbelkeilanordnung nach Anspruch 6, **dadurch gekennzeichnet, dass** sie ferner ein Verbindungselement (190) umfasst, das die Keile miteinander verbindet.

8. Zwischenwirbelkeilanordnung nach Anspruch 7, **dadurch gekennzeichnet, dass** die Form des Verbindungselements (190) so gewählt ist, dass dieses Element die Dornfortsätze (A2, A1) der Wirbel V1, V2) umgibt.

9. Zwischenwirbelkeilanordnung nach Anspruch 8, **dadurch gekennzeichnet, dass** das Verbindungselement (190) die allgemeine Form eines V hat und zwei gekrümmte Schenkel (192, 194) aufweist, die voneinander beabstandet sind und deren freie Enden an dem ersten und zweiten Keil befestigt sind, wobei die Länge der Schenkel, ihre Krümmung und ihr Abstandswinkel (δ) so gewählt sind, dass das Verbindungselement die Dornfortsätze (A2, A1) der Wirbel umgibt.

10. Zwischenwirbelkeilanordnung nach Anspruch 9, **dadurch gekennzeichnet, dass** der Abstand (L) zwischen den freien Enden der Schenkel (192, 194) und dem Verbindungspunkt (193) der Schenkel zwischen 10 und 40 mm beträgt und dass der Abstandswinkel (δ) der Schenkel zwischen 10 und 60° beträgt.

## Claims

1. Intervertebral wedge intended to be placed between two adjacent, upper and lower, cervical vertebrae (V2, V1), comprising a wedge body (10) and means for holding said wedge body (10) in position against said vertebrae, said wedge body comprising:
- a spacer part (12) having opposing first and second ends (12a, 12b), a portion of the first end (12a) being capable of being inserted between the top edge of the lamina of the lower cervical vertebra (V1) and the bottom edge of the lamina of the upper cervical vertebra (V2);
- two, upper and lower, flanges (16, 14) connected by the base thereof to the second end (12b) of the spacer part (12) and extending from each side thereof, each flange having a front face (16a, 14a) facing towards the spacer part (12) and a rear face (16b, 14b), the front faces (16a, 14a) of the upper and lower flanges being capable of coming to bear against the rear portions of the laminae of the upper and lower vertebrae (V2, V1), respectively;
- **characterised in that** said means for holding the wedge body in position comprise a strap (60; 160) having two ends (60a, 60b; 160a, 160b) and self-locking fastener means (70b, 170) allowing at least one of the ends of the strap to be rigidly connected to the wedge body (10), said strap (60; 160) being intended to be tightened around the laminae of the upper and lower vertebrae (V2, V1).

2. Intervertebral wedge according to claim 1, **characterised in that** said means for holding the wedge body in position comprise first fastener means (70a) arranged on one of the flanges of the wedge body (10) for securing a first end (60a) of said strap (60) to the wedge body, and self-locking second fastener means (70b) arranged on the other flange of the wedge body, the second end (60b) of the strap being passed therethrough and then pulled in order to tighten the strap around the laminae of the upper and lower vertebrae (V2, V1), the second fastener means (70b) being made in such a manner as to produce frictional forces that oppose loosening of said strap (60).

3. Intervertebral wedge according to claim 1, **characterised in that** said self-locking fastener means (170) are arranged on the rear face of the upper flange (16) and/or of the lower flange (14), the first and second ends of the strap (160a, 160b) being passed through said fastener means (170) and then pulled in order to tighten the strap around the laminae of the upper and lower vertebrae (V2, V1), said self-locking fastener means being made in such a manner as to produce frictional forces that oppose loosening of said strap (160).

4. Intervertebral wedge according to any of claims 1 to 3, **characterised in that** the rear faces (16b, 14b) of the upper and lower flanges are coplanar.

5. Intervertebral wedge according to any of claims 1 to 4, **characterised in that** said self-locking fastener means (70b, 170) are removable from the wedge body (10) and are fitted on the rear face of the upper flange (16) and/or of the lower flange (14).

6. Set of intervertebral wedges, **characterised in that** it comprises a first and a second intervertebral wedge according to any of the preceding claims, the first wedge being intended to be arranged between the laminae of the upper and lower cervical vertebrae (V2, V1) arranged on one side of the spinous processes (A2, A1) of said vertebrae, and the second wedge being intended to be arranged between the laminae of the upper and lower cervical vertebrae arranged on the other side of said spinous processes (A2, A1).

7. Set of intervertebral wedges according to claim 6, **characterised in that** it further comprises a connection element (190) interconnecting the wedges.

8. Set of intervertebral wedges according to claim 7, **characterised in that** the shape of said connection element (190) is selected in such a manner that said element goes around the spinous processes (A2, A1) of said vertebrae (V2, V1).

9. Set of intervertebral wedges according to claim 8, **characterised in that** said connection element (190) is generally V-shaped and has two curved limbs (192, 194) that are spaced apart from each other, the free ends of which are secured to the first and second wedges, the length of the limbs, their curvature, and their spacing angle (δ) being selected in such a manner that the junction element goes around the spinous processes (A2, A1) of said vertebrae.

10. Set of intervertebral wedges according to claim 9, **characterised in that** the distance (L) between the free ends of the limbs (192, 194) and the junction point (193) between said limbs is between 10 mm and 40 mm, and **in that** said spacing angle (0) between said limbs is between 10 and 60°.
